(19) Europäisches Patentamt
European Patent Office
Office européen des brevets

(11) Veröffentlichungsnummer: **0 152 856**
**A2**

(12) # EUROPÄISCHE PATENTANMELDUNG

(21) Anmeldenummer: **85101165.0**

(22) Anmeldetag: **05.02.85**

(51) Int. Cl.⁴: **C 07 H 7/02**, C 07 H 13/04, C 07 H 13/12, C 07 H 13/06, C 07 H 15/12, A 61 K 31/70

(30) Priorität: **17.02.84 DE 3405841**

(43) Veröffentlichungstag der Anmeldung: **28.08.85 Patentblatt 85/35**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Lockhoff, Oswald, Dr., Morgengraben 14, D-5000 Köln 80 (DE)**
Erfinder: **Krüger, Bernd-Wieland, Dr., Sillerstrasse 49, D-5600 Wuppertal 1 (DE)**
Erfinder: **Stadler, Peter, Dr., Am Ideck 8, D-5657 Haan 1 (DE)**
Erfinder: **Metzger, Karl Georg, Dr., Pahlkestrasse 75, D-5600 Wuppertal 1 (DE)**
Erfinder: **Kroll, Hein-Peter, Dr., Pahlkestrasse 96, D-5600 Wuppertal 1 (DE)**
Erfinder: **Schaller, Klaus, Dr., Am Sonnenschein 38, D-5600 Wuppertal 1 (DE)**
Erfinder: **Zeiler, Hans-Joachim, Dr., Elsbeeker Strasse 46, D-5620 Velbert 15 (DE)**

(54) **N-acylierte 1-Alkylamino-1-desoxy-ketose-Derivate, Verfahren zur Herstellung und ihre Verwendung.**

(57) Die vorliegende Erfindung betrifft neue N-acylierte 1-Alkylamino-1-desoxy-Ketosederivate der allgemeinen Formel I, in welcher $R^1$, X und $R^2$ die in der Beschreibung angegebene Bedeutung besitzen, Verfahren zu ihrer Herstellung sowie ihre Verwendung als Arzneimittel.

EP 0 152 856 A2

BAYER AKTIENGESELLSCHAFT 5090 Leverkusen, Bayerwerk

Konzernverwaltung RP
Patentabteilung Ad/ABc

## N-acylierte 1-Alkylamino-1-desoxy-ketose-Derivate, Verfahren zur Herstellung und ihre Verwendung

Die Erfindung betrifft neue N-acylierte 1-Alkylamino-1-desoxy-Ketosederivate, Verfahren zu ihrer Herstellung sowie ihre Verwendung als Arzneimittel.

Die neuen Verbindungen entsprechen den Formeln Ia bis Ic, die nachfolgend zusammenfassend als Formel I bezeichnet werden.

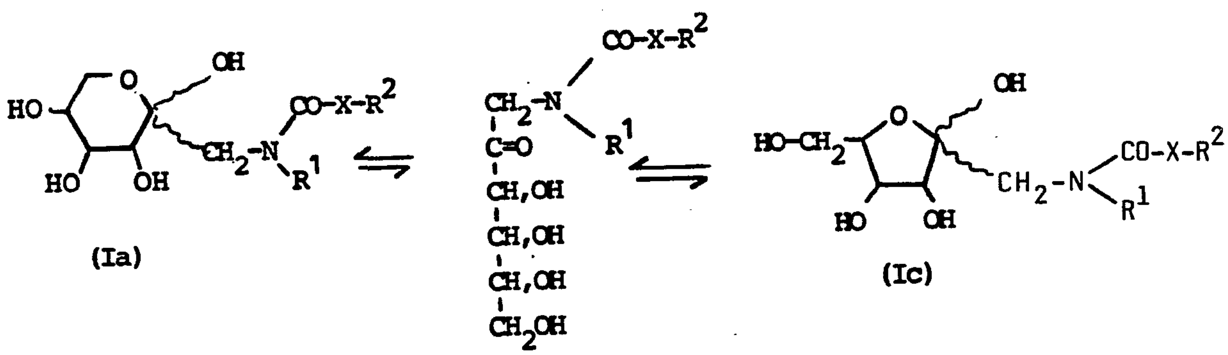

(Ia) (Ib) (Ic)

Der Zuckerrest in Formel I ist ein N-subtituierter 1-Amino-1-desoxy-Ketozucker, wobei die Ketogruppe am Kohlenstoffatom C-2 angeordnet ist. Gewöhnlich bildet diese Ketofunktion unter Einbeziehung einer Hydroxygruppe ein cyclisches Halbketal. Dieses Halbketal steht mit seiner offenkettigen Form im Gleichgewicht, das Gleichgewicht liegt aber deutlich auf der Seite der Ringform.

Wird ein Halbketal mit der Hydroxygruppe gebildet, die an C-6 des Zuckers angeordnet ist, so bildet sich ein sauerstoffhaltiger Sechsring aus, wie in Formel Ia gezeigt. Bildet sich das Halbketal mit der Hydroxylgruppe an C-5 des Zuckers aus, entsteht ein sauerstoffhaltiger Fünfring der Formel Ic. Die offenkettige Form des Zuckers hat die Formel Ib. Da die beiden cyclischen Formeln gemäß Formel Ia und Formel Ic mit der offenkettigen Form gemäß Formel Ib untereinander im Gleichgewicht stehen, werden die neuen Verbindungen zutreffend durch die Formeln Ia bis Ic charakterisiert.

Hierin stellt:

$R^1$ Wasserstoff oder einen gegebenenfalls substituierten, geradkettigen oder verzweigten Alkyl- oder ein- oder mehrfach ungesättigten Alkenylrest mit jeweils bis zu 30 C-Atomen dar,

X steht für $CH_2$, O, S oder NR, worin R Wasserstoff oder eine Alkylgruppe mit bis zu 20 C-Atomen bedeutet und

$R^2$ stellt Wasserstoff oder einen gegebenenfalls substituierten, geradkettigen oder verzweigten, gesättigten oder ein- oder mehrfach ungesättigten Kohlenwasserstoffrest mit bis zu 30 C-Atomen dar.

Bevorzugt stellt $R^1$ einen Alkylrest mit einem bis 21 Kohlenstoffatomen, vorzugsweise 7 bis 21 C-Atomen dar. Beispielhaft für diese Reste $R^1$ seien hier genannt: Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Didecyl, Methyldecyl, Methyloctadecyl, Decylhexadecyl.

Geeignete Alkenylreste sind beispielsweise Ethenyl, Propenyl-1, Propenyl-2, i-Butenyl, Butenyl-1, Butenyl-2, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Decenyl, 5-Decenyl,9-Decenyl, 8-Heptadecenyl, 1,3-Butadienyl, 1,3-Pentadienyl, 1,4-Pentadienyl, 2,4-Pentadienyl, 8,11-Heptadecandienyl, 8,11,14-Heptadecantrienyl. Im allgemeinen sind die längerkettigen, ungesättigten Reste bevorzugt, speziell die ein- oder zweifach ungesättigten Alkenyle mit 7-21 C-Atomen.

Die ungesättigten Kohlenwasserstoffreste können dabei als reine cis- oder trans-Isomere oder auch als Isomerengemische vorliegen.

Unter den Alkyl- und Alkenylresten $R^1$ werden erfindungsgemäß auch solche Reste verstanden, in denen eine oder mehrere, vorzugsweise eine oder zwei Methylen- oder Methingruppen durch Sauerstoff, Schwefel und/oder Stickstoff ersetzt sein können. Bei einer Unterbrechung der Kette durch Stickstoffatome trägt dieses N entweder ein Wasserstoffatom oder einen $C_1$-$C_{30}$-, vorzugsweise $C_1$-$C_{10}$-Alkylrest oder einen -CO-Alkylrest mit bis zu 25, vorzugsweise 1 bis 10 Kohlenstoffatomen.

Beispiele für Fälle in denen die Reste $R_1$ durch O, S und N bzw. entsprechende Atomgruppierungen unterbrochen sind, sind Methoxyethyl, Methoxyethoxyethyl, Ethylthiododecyl und N-Methyl-aminodecyl.

Als Substituenten für den Rest $R^1$ kommen folgende bevorzugt in Frage:

Aryl, vorzugsweise Phenyl; Halogen, vorzugsweise Fluor, Chlor und Brom; Amino, $C_1$-$C_6$-Alkylamino und Di-$C_1$-$C_6$-alkylamino; OH; $C_1$-$C_6$-Alkoxy; SH; $C_1$-$C_6$-Alkyl-COO und $C_1$-$C_6$-Alkyl-CO-NH.

Wenn $R^1$ substituiert ist, so liegen im allgemeinen 1-5, vorzugsweise 1-3 Substituenten vor.

Beispiele für substituierte Reste $R^1$ sind Hydroxyheptadecenyl, Aminodecyl, Fluormethyl-ß-hydroxytridecyl, Mercaptoethyl,Phenyltetradecyl, Fluortetradecyl, Fluorhexadecyl, Fluoroctadecyl, Chlordodecyl, Chlorpentadecyl, Chlorhexadecyl, Bromoctadecyl, Aminododecyl, Aminohexadecyl, Ethylamino-hexadecyl, N,N-Dihexylaminododecyl, Hydroxydodecyl, Hydroxytetradecyl, Hydroxyhexadedecyl, Methoxytetradecyl, Butoxytetradecyl, Acetyloxyhexadecyl, Acetylamidotetradecyl, Myristoyloxytetradecyl und Myristoylamidohexadecyl.

Der Kohlenwasserstoffrest $R^2$ kann erfindungsgemäß ein Alkyl-, Alkenyl-, Cycloalkyl-, Cycloalkenyl- oder Arylrest sein, wobei diese auch gemeinsam auftreten können, z.B. als Alkylcycloalkyl, Aralkyl oder Alkylaryl.

Der Rest $R^2$ hat vorzugsweise 1-20 C-Atome. Unter Kohlenwasserstoffresten $R^2$ werden erfindungsgemäß auch solche verstanden, bei denen bis zu 5, vorzugsweise eine oder zwei Methylen- oder Methingruppen durch O, S und/oder NR ersetzt sind, worin R für Wasserstoff oder $C_1$-$C_6$, vorzugsweise $C_1$-$C_3$-Alkyl steht.

Beispiele, in welchen $R^2$ für einen Alkylrest oder Alkenylrest steht die für $R^1$ genannten.

Besonders seien genannt: Methyl, Propyl, Hexyl, Decyl, Undecyl, Dodecyl, Tridecyl, Tetradecyl, Pentadecyl, Hexadecyl, Heptadecyl, Octadecyl, Nonadecyl, Docosyl, Myricyl, Ethylhexyl, Isobutyl, Propenyl, Octenyl, Hexadienyl, Docosenyl und Dimethylhexenyl. Die ungesättigten Kohlenwasserstoffrest können als reine cis- oder trans-Isomere oder als Isomerengemisch vorliegen.

Beispiele für $R^2$ in der Bedeutung Cycloalkyl sind Cyclopropyl, Cyclobutyl, Cyclopentyl, Cyclohexyl, Cycloheptyl und Cyclooctyl. Die Ringe mit 5, 6 und 7 C-Atomen sind bevorzugt.

Cycloalkenylreste $R^2$ sind beispielsweise Cyclopentenyl, Cyclopentadienyl, Cyclohexenyl, Cyclohexadienyl und Cycloheptenyl.

$R^2$ in der Bedeutung Aryl steht vorzugsweise für aromatische Reste mit 6, 10 oder 12 C-Atomen. Hier sind Phenyl, Naphthyl und der Diphenylrest besonders zu nennen.

Die Reste $R^2$ können ein- oder mehrfach, im allgemeinen 1-5 fach, vorzugsweise 1-3 fach substituiert sein. Als Substituenten kommen die für $R^1$ genannten Substituenten in Betracht.

Beispiele für substituierte Alkylreste $R^2$ sind Methyltetradecyl, Hydroxytridecyl, Hydroxyoctadecyl, Chlortridecyl, Acetyloxytridecyl, Myristoyloxytridecyl, Aminotridecyl, Acetylamidotridecyl, Mercaptooctadecyl.

Beispiele für substituierte Alkenylreste $R^2$ sind Hydroxytetradecenyl, Acetamidooctadecenyl, Methoxyheptadecenyl.

Als Beispiele für substituierte Cycloalkyl- und Cycloalkenylreste können angeführt werden, Chlorcyclohexyl, Methoxycycloheptyl, Hydroxycyclohexenyl, Acetamidocyclohexenyl.

Beispiele von Substituenten an aromatischen Resten $R^2$ sind p-Nitrophenyl, 2,4-Dichlorphenyl, p-Methoxyphenyl, Trifluormethylphenyl, Dimethylaminophenyl.

Weitere Beispiele für Reste $R^2$, in denen $CH_2$- oder CH-Gruppen wie angegeben ersetzt sind, sind folgende: $\omega$-(N-Ethyl-N-butyl)-aminodedecyl, $\omega$-Piperidinyltetradecyl, $\omega$-Butoxyoctadecyl, $\omega$-Morpholinoheptadecyl.

Schließlich seien als Beispiele für das gemeinsame Auftreten der Bedeutungen von $R^2$ folgende genannt: 2-(Cyclohexyl)ethyl, Benzyl, Phenethyl, Phenylhexyl, Decahydronaphthylethyl, Butyldecahydronaphthyl.

Wenn X in Formel I eine N-Alkylgruppe darstellt, so hat diese Alkylgruppe vorzugsweise 1-10 C-Atome.

Die Verbindungen der Formel I enthalten mehrere chirale Kohlenstoffatome und liegen als optisch reine Diastereomere oder als Diastereomerengemische vor.

Beispiele für die durch Formel I beschriebenen Zuckerderivate sind also N-substituierte 1-Amino-1-desoxy-Derivate der Psicose, Fructose, Sorbose oder Tagatose.

Die Erfindung betrifft auch Verfahren zur Herstellung der Verbindungen gemäß Formel I. Hierzu setzt man Hexosen wie z.B. Allose, Altrose, Glucose, Mannose, Gulose, Idose, Galactose oder Talose entweder in freier, d.h. ungeschützter Form, oder in Form geschützter, gegebenenfalls aktivierter Derivate um mit einer Aminoverbindung $R^1$-$NH_2$, entweder in freier Form oder in Form eines geeigneten Säureadditionssalzes, wobei $R^1$ die vorstehend beschriebene Bedeutung hat. Das durch diesen Schritt erhaltene Glycosylamin wird in einem zweiten Schritt, entweder ohne oder aber auch mit geeigneten Katalysatoren, umgelagert in die N-Alkyl-1-amino-1-desoxy-Ketosen.

Diese aminohaltigen Ketozucker werden dann in einem dritten Reaktionsschritt mit einem gegebenenfalls aktivierten Carbonsäure-, Thiokohlensäure-, Halogenameisensäureester- oder Isocyanatderivat umgesetzt, gegebenenfalls unter Zusatz von Basen. Harnstoffderivate erhält man auch durch Umsetzung von aromatischen Carbamaten mit primären oder sekundären Aminen. Von den auf diese Weise erhaltenen Carbonsäureamiden, Carbamaten, Thiocarbamaten oder Harnstoffen spaltet man dann gegebenenfalls vorhandene Schutzgruppen ab und erhält auf diese Weise die erfindungsgemäßen Verbindungen der Formel I, die, falls erforderlich, durch Chromatographie, Umkristallisieren, Extraktion o.ä. gereinigt werden können.

In einer bevorzugten Ausführungsform des erfindungsgemäßen Verfahrens wird in an sich bekannter Weise in einem ersten Verfahrensschritt die unblockierte Hexose in einem geeigneten Lösungsmittel, gegebenenfalls in Gegenwart eines Katalysators bei Temperaturen zwischen 0°C und 80°C mit 1 bis 10 Äquivalenten des betreffenden Amins $R^1$-$NH_2$ umgesetzt. Dabei erhält man gewöhnlich in hohen Ausbeuten die betreffenden Glycosylamine der Formel II

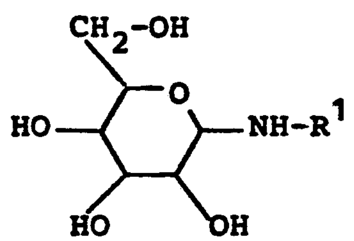

(II)

die als amorphe oder kristalline Feststoffe oder als zähe Sirupe anfallen.

Im zweiten Verfahrensschritt wird das Glycosylamin der Formel II in einem geeigneten Lösungsmittel entweder durch langes Erhitzen ohne Katalysator oder in Gegenwart einer organischen oder anorganischen Säure umgelagert in eine N-alkylierte 1-Amino-1-desoxy-Ketose der Formel III

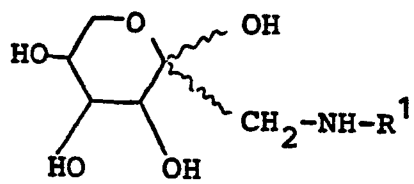

(III)

Umlagerungen dieses Typs sind seit langem bekannt und haben unter dem Namen "Amadori-Umlagerung" Eingang in die chemische Literatur gefunden.

In einem dritten Verfahrensschritt wird die Aminoketose der Formel III umgesetzt zu Verbindungen der Formeln Ia - Ic.

Für den Fall, daß X in Formel I eine Methylengruppe darstellt, setzt man die entsprechenden Amine der Formel III um mit 1 bis 10 Äquivalenten eines Carbonsäurederivats der Formel $R^2-CH_2-CO-Y$, in der $R^2$ die oben angegebene Bedeutung besitzt und Y Halogen oder eine bei Amidierungen übliche Abgangsgruppe bedeutet, vorzugsweise einen aktivierenden Esterrest oder eine Gruppe $O-CO-R^2$ mit

der obigen Bedeutung für $R^2$, oder eine Gruppe O-CO-O-$R^2$ mit der obigen Bedeutung für $R^2$. Dabei arbeitet man in organischen oder wäßrig-organischen Lösungsmitteln bei Temperaturen zwischen 0° und 50°C, gegebenenfalls in Gegenwart einer Base.

Für den Fall, daß X in Formel I ein Sauerstoffatom darstellt, setzt man ein Amin der Formel III um mit 1 bis 5 Äquivalenten eines Halogenkohlensäureesters Y-CO-O-$R^2$, wobei Y für Halogen, bevorzugt Chlor, steht und $R^2$ die oben beschriebene Bedeutung besitzt. Man arbeitet dabei in organischen Lösungsmitteln bei einer Temperatur zwischen -20°C und 50°C, gegebenenfalls in Gegenwart einer Base.

Für den Fall, daß X in Formel I eine NH-Gruppe bedeutet, setzt man ein Amin der Formel III um mit 1 bis 5 Äquivalenten eines Isocyanats $R^2$-NCO mit der obengenannten Bedeutung von $R^2$. Es wird bevorzugt in organischen Lösungsmitteln umgesetzt, gegebenenfalls in Gegenwart eines Katalysators, wobei die Temperaturen zwischen -20°C und 50°C liegen.

Für den Fall, daß X in Formel I eine NH- oder N-Alkylgruppe bedeutet, kann man auch ein Carbamat, das man durch Reaktion des Amins gemäß Formel II mit einem bevorzugt aromatischen Halogenameisensäureesters erhält, mit 1 bis 10 Äquivalenten eines primären Amins $R^2$-$NH_2$ oder eines sekundären Amins $R^2$-NH-Alkyl mit den vorstehend beschriebenen Bedeutungen für $R^2$ zur Reaktion bringen. Es wird

bevorzugt bei 20°C bis 80°C in organischen Lösungsmitteln umgesetzt.

Für den Fall, daß X in Formel I ein Schwefelatom darstellt, läßt man ein Amin der Formel III reagieren mit einem Thiokohlensäurechlorid-S-ester $R^2$-S-CO-Y, wobei Y für Halogen, bevorzugt Chlor, steht und $R^2$ die obige Bedeutung zukommt. Es wird in organischen Lösungsmitteln gearbeitet, gegebenenfalls in Gegenwart eines geeigneten Katalysators und/oder einer Base, die Reaktionstemperaturen liegen zwischen 0° und 70°C.

In allen beschriebenen Fällen, die zu Verbindungen der Formel I führen mit den oben genannten Bedeutungen für X wird das Reaktionsprodukt in üblicher Weise aufgearbeitet.

Der erste Verfahrensschritt bei der Herstellung der erfindungsgemäßen Verbindungen der Formel I ist somit die Umsetzung eines Zuckers, der am anomeren Kohlenstoffatom unblockiert ist, mit einem Amin des Typs $R^1$-$NH_2$ am anomeren Kohlenstoffatom unter Wasserabspaltung zu dem betreffenden Glykosylamin.

Amine $R^1$-$NH_2$, die bei Raumtemperatur flüssig sind, können mit dem Zucker direkt, d.h. ohne Lösungsmittel umgesetzt werden. Hierbei arbeitet man bei Temperaturen zwischen 0°C und 100°C vorzugsweise bei 25°C bis 70°C. Als Katalysatoren sind Mineralsäuren wie z.B. Salzsäure, Schwefelsäure oder Salpetersäure oder kurzkettige

Carbonsäuren wie Essigsäure oder Propionsäure geeignet, die man in Mengen von 0,001 bis 0,5 Äquivalenten einsetzt.

In jedem Fall ist es möglich, und bei Aminen $R^1$-$NH_2$, die bei Raumtemperatur fest sind auch zu bevorzugen, die Herstellung der Glykosylamine in Gegenwart eines Lösungsmittels durchzuführen. Man arbeitet dann vorzugsweise in Gegenwart eines unter den Reaktionsbedingungen inerten Verdünnungsmittels, welches vorzugsweise so beschaffen ist, daß sich zumindest entweder die Reaktionspartner oder das Reaktionsprodukt in ihm lösen.

In Frage kommen Alkohole wie Methanol, Ethanol, Propanol-1 und Propanol-2, Ether wie Tetrahydrofuran und Dioxan, sowie auch Dimethylformamid, wobei - außer bei der Verwendung der Alkohole - der Zusatz von Wasser zu bevorzugen ist. Darüber hinaus ist vorzugsweise bei kurzkettigen Aminen $R^1$-$NH_2$ auch Wasser allein als Lösungsmittel geeignet. Es kann auch von Vorteil sein, die Alkanole im Gemisch mit Wasser zu verwenden.

Die Reaktionstemperaturen bei Verwendung von Lösungsmitteln bei der Herstellung der Glykosylamine liegen zwischen -10°C und 120°C vorzugsweise zwischen 30°C und 70°C.

Das betreffende Verdünnungsmittel kann wahlweise vor oder während der Reaktion zugegeben werden. Bei lang-

kettigen Aminen $R^1-NH_2$ ist eine Zugabe vor der Reaktion zu bevorzugen.

Die wie vorstehend beschriebenen hergestellten Glykosylamine kristallisieren entweder direkt oder nach Abkühlen aus und können durch Zusatz geeigneter, vorzugsweise weniger polarer Hilfslösungsmittel wie Aceton, Diethylether, Cyclohexan, Essigester oder Petrolether, gegebenenfalls unter Kühlung, ausgefällt oder zur Kristallisation gebracht werden, gegebenenfalls vorhandenes überschüssiges Amin $R^1-NH_2$ kann durch Waschen oder Umkristallisieren des Produktes auf an sich bekannte Weise entfernt werden.

Der zweite Verfahrensschritt besteht in der Umlagerung des N-substituierten Glycosylamins der Formel II in die N-substituierte 1-Amino-1-desoxy-2-Ketose der Formel III. Diese Reaktion läßt sich ohne oder mit einem Lösungsmittel durchführen. Wird die Reaktion ohne Lösungsmittel durchgeführt, so arbeitet man bei erhöhter Temperatur, zwischen 50°C und 150°C vorzugsweise bei 100°C bis 120°C, zumindest aber oberhalb des Schmelzpunktes der eingesetzten Glycosylamine. Durch Zusatz von Mineralsäuren oder organischen Säuren, wie z.B. Essigsäure oder Oxalsäure, läßt sich die Ausbeute an gewünschtem Umlagerungsprodukt der Formel III erhöhen. Die Säuremenge bewegt sich im Bereich von 0,0001 bis 0,5 Moläquivalenten, bevorzugt 0,001 bis 0,1 Moläquivalenten, bezogen auf das Glycosylamin der Formel II.

Es ist jedoch im allgemeinen zu bevorzugen, die Umlagerung in einem organischen und bevorzugt wasserfreien Lösungsmittel durchzuführen. Geeignete Lösungsmittel sind z.B. Methanol, Ethanol, Propanol, Isopropanol, Tetrahydrofuran, Dioxan oder Dimethylformamid. Auch bei dieser Variante ist es vorteilhaft, die Umlagerung durch Mineralsäuren oder die oben genannten organischen Säuren zu katalysieren.

Pro Mol Aldosylamin der Formel II werden etwa 0,02 bis 0,5 Mol der Säure zugesetzt und das Gemisch für einige Zeit, vorzugsweise 10 bis 100 min, erwärmt. Die Reaktionstemperaturen bewegt sich im Bereich von 40°C bis 150°C, bevorzugt zwischen 60°C und 90°C.

Es ist auch möglich, die Umlagerung in dem organischen Lösungsmittel durch Verbindungen mit aktiven Methylengruppierungen zu katalysieren.

Geeignete Verbindungen dieser Art sind z.B. Ethylmalonat, 2,4-Pentandion, Phenylaceton, Diphenylmethan oder Malonsäure.

Es ist ferner möglich und z.T. auch zu bevorzugen, die oben genannten Verfahrensschritte eins und zwei, d.h. die Darstellung des Aldosylamins der Formel II und dessen Umlagerung zur substituierten 1-Amino-1-desoxy-2-ketose der Formel III, in einem Reaktionsschritt zusammen zu fassen. Bei dieser Verfahrensvariante werden die Aldose und das Amin $R^1-NH_2$ direkt, entweder ohne

oder aber mit einem der oben genannten Lösungsmittel unter der Katalyse von Mineralsäuren oder den oben genannten organischen Säuren zur Reaktion gebracht. Diese Variante ist in der Literatur beschrieben (HODGE, Advances in Carbohydrate Chemistry, 10 (1955) 169).

In den meisten Fällen ist die Umlagerung von Substanzen der Formel II zu Substanzen der Formel III begleitet durch eine mehr oder weniger starke Zersetzung der Zuckerkomponente, die verstärkt auftritt bei zu langen Heizzeiten, zu hohen Temperaturen oder zu hohen Säurekonzentrationen und sich in der Dunkelfärbung des Reaktionsansatzes äußert.

In jedem Fall ist deshalb nach der Synthese der N-substituierten 1-Amino-1-desoxy-Ketosen der Formel III eine Aufreinigung des Reaktionsansatzes anzuschließen. Die Verbindungen der Formel III werden deshalb durch übliche Reinigungsschritte wie Kristallisation, Extraktion, Chromatographie o.a. aus den Ansätzen isoliert.

Der dritte Verfahrensschritt bei der Herstellung der erfindungsgemäßen Verbindungen der Formel I ist die selektive Umsetzung der N-substituierten 1-Amino-1-desoxy-2-ketosen der Formel III an der Aminofunktion mit einem Carbonsäurederivat $R^2-CH_2-CO-Y$ mit der vorstehend angegebenen Bedeutung von $R^2$ und Y, oder mit einem Halogenkohlensäureester $Y-CO-O-R^2$ mit der vorstehend angegebenen Bedeutung von $R^2$ und Y, oder mit einem Isocyanat $R^2-NCO$ mit der vorstehenden Bedeutung von $R^2$, oder mit einem

Thiokohlensäureester-S-chlorid $R^2$-S-CO-Y mit der vorstehenden Bedeutung von $R^2$ und Y, oder nacheinander durchgeführt mit bevorzugt aromatischen Halogenkohlensäureestern Y-CO-O-Ar mit der oben bei $R^2$ beschriebenen Bedeutung für Y und den aromatischen Rest zum Carbamat, gefolgt von der Aminolyse mit primären oder sekundären Aminen $R^2$-$NH_2$ oder $R^2$-NH-Alkyl zum Harnstoffderivat.

Diese Carbonsäure- bzw. Kohlensäurederivate werden vorzugsweise in Gegenwart eines Verdünnungsmittels mit den 1-Amino-1-desoxy-Ketosederivaten umsetzt, in welchem die Reaktionspartner vollständig oder auch nur teilweise gelöst sind.

Es kommen organische oder anorganische Solventien in Frage, vorzugsweise solche, die unter den Reaktionsbedingungen Nebenreaktionen möglichst herabsetzen oder verhindern. Man kann sowohl in organischen Lösungsmitteln wie Ethern, z.B. Tetrahydrofuran und Dioxan, oder Alkoholen, z.B. Ethanol und Propanol, oder Ketonen, z.B. Aceton und Methylethylketon, oder in Dimethylformamid, Essigester oder Pyridin oder auch in Mischungen dieser Lösungsmittel untereinander und/oder mit Wasser arbeiten. Die Verwendung von wasserfreien Solventien ist im allgemeinen zu bevorzugen.

Die Carbonsäurederivate $R^2$-$CH_2$-CO-Y bzw. die Kohlensäurederivate $R^2$-O-CO-Y, $R^2$-NCO und $R^2$-S-CO-Y werden in 1 bis 10 Äquivalenten, bezogen auf das Ketosederivat der Formel III eingesetzt, wobei die Verwendung von 1 bis 3 Äquivalenten zu bevorzugen ist.

Die Acylierungsreaktionen können in Gegenwart basischer Hilfsstoffe durchgeführt werden. Verwendet werden können alle in der organischen Synthese üblichen basischen Verbindungen wie z.B. tertiäre aliphatische oder auch aromatische Amine oder aber Alkali- oder Erdalkalihydroxide bzw. -carbonate wie Natronlauge, Natriumcarbonat oder Calciumcarbonat oder aber Anionenaustauscherharze oder Kationenaustauscherharze.

Die Umsetzungen mit den Carbonsäure- bzw. Kohlensäurederivaten werden bei Temperaturen zwischen etwa -30°C und +80°C, vorzugsweise zwischen -10°C und +25°C durchgeführt.

Die auf diese Weise erhaltenen Amide, Carbamate, Harnstoffe oder Thiocarbamate gemäß Formel I werden nach an sich bekannten Verfahren in Form von kristallinen oder amorphen Feststoffen oder als Sirupe isoliert und, wenn notwendig, durch Umkristallisation, Chromatographie, Extraktion usw. gereinigt.

Zum Gegenstand der Erfindung gehören auch Salze der Verbindungen der Formel I. Dabei handelt es sich in erster Linie um üblicherweise pharmazeutisch verwendbare, nicht toxische Salze.

Die Verbindungen der Erfindung weisen eine Abwehr-steigende Wirkung auf. Es wurde gefunden, daß die Verbindungsklasse die unspezifische wirtseigene Abwehr verstärkt. Diese Ergebnisse wurden anhand der nachfolgenden Versuchsanordnungen erhalten.

Versuchsbeschreibung

Mäuse vom Typ SPF-CFW1 wurden intravenös mit 2-6x $10^5$ logarithmisch wachsenden Zellen von Candida albicans, suspendiert in physiologischer Kochsalzlösung, infiziert. Beginnend mit dem 3. Tag post infectionem werden bei unbehandelten Kontrolltieren die ersten Krankheitssymtome erkennbar. Bis zum 5. Tag sterben die ersten Tiere an akutem Nierenversagen und bis zum 14.Tag post infectionem sind in der Regel mehr als 80 % der unbehandelten Tiere gestorben. In diesem Test zeigen sich die Verbindungen der Beispiele 9, 15, 16 und 28 als krankheitsverzögernd wirksam. Eine signifikante krankheitsverzögernde Wirkung wurde erreicht, wenn die Substanz jeweils einmal 24 Stunden von der Infektion in Konzentrationen von 1-50 mg/kg Körpergewicht intraperitoneal (i.p.) verabreicht wurde.

Bei behandelten Tieren wurde eine statistisch signifikante Verlängerung der Überlebenszeit im Vergleich zu den unbehandelten Kontrollen beobachtet. Etwa 50 % der behandelten Tiere überlebten einen Beobachtungszeitraum von 14 Tagen, verglichen mit etwa 20 % unbehandelter Kontrolltiere.

Die erfindungsgemäßen Verbindungen können allein als Prophylaktikum, zur Behandlung bestehender Infektionen oder in Kombinationen mit einer antibiotischen Therapie zur Steigerung der therapeutischen Wirkung von Antibiotika und Chemotherapeutika (z.B. Penicilline, Cephalosporine,

Aminoglykoside etc.) bei infizierten Menschen und Tieren verwendet werden.

Es wurde gefunden, daß Infektionen der Maus mit pathogenen Keimen, die innerhalb von 24-48 Stunden zum Tod der Versuchstiere führen, durch eine prophylaktische Behandlung - bevorzugt intraperitoneal - mit 1-80 mg/kg der erfindungsgemäßen Verbindungen therapiert werden können. Dies trifft für eine ganze Reihe grampositiver (z.B. Staphylokokken) und gramnegativer (z.B. E.coli, Klebsiella, Proteus, Pseudomonas) Krankheitserreger zu. Diese Aufzählung ist beispielhaft und keineswegs beschränkend aufzufassen. So überleben z.B. Mäuse, die mit dem pathogenen Stamm Klebsiella 63 infiziert worden waren, nach Behandlung (z.B. 18 Stunden vor Infektion) mit 10-40 mg/kg der erfindungsgemäßen Verbindungen der Beispiele 9, 13, 15, 24, 27, 28, zu 40-100 % diese Infektion, während von den unbehandelten Kontrolltieren nur 0-30 % überlebten.

In einem weiteren Versuchsmodell konnte gezeigt werden, daß die therapeutische Wirksamkeit von Antibiotika durch die erfindungsgemäßen Verbindungen gesteigert werden kann. So wurde Mäuse mit dem Stamm Pseudomonas W. infiziert. Diese Infektion führte bei den meisten Kontrolltieren innerhalb 24 Stunden zum Tode. Eine weitere Gruppe wurde mit 4 mg/kg Sisomicin 30 Stunden post infektionem behandelt. Es konnte gezeigt werden, daß in der Versuchsgruppe, die mit den erfindungsgemäßen Verbindungen der Beispiele 9, 13, 15, 24 18 Stunden vor Infektion behandelt worden waren, die therapeutische Wirksamkeit des Sisomicins entscheidend verbessert werden kann.

Salmonella typhimurium - Infektion der Maus

Die Versuchstiere, $CFW_1$ Mäuse ♀♀ ca. 20 g Körpergewicht, wurden randomisiert in Gruppen à 10 Mäusen pro Käfig eingesetzt. Die Gruppen wurden mit jeweils 0,5 ml der in Glucose-Agar formulierten Substanzen in den Kontrollgruppen mit der Leerformulierung (ohne Substanz), jeweils 3 mal behandelt. Die Behandlung erfolgte 24 Stunden und 1 Stunde vor Infektion und 24 Stunden nach Infektion. Die i.p.-Infektion mit Salmonella typhimurium Stamm LT2 und ca. $5x10^5$ Keimen/Maus in 0,25 ml entsprach einer LD50. Der Infektionsverlauf der Kontrollgruppen zeichnete sich durch eine Anfangsphase von 4 Tagen aus, an denen die Tiere nicht starben. Diese Anfangsphase bietet den Tieren die Möglichkeit, zelluläre Immunmechanismen zu aktivieren und simuliert somit die unspezifische Abwehr einer latenten oder chronischen Infektion. Vom Tag 4 bis 12 nach Infektion starben ca. 50 % der Kontrolltiere. Nach einer Beobachtungszeit von 21 Tagen wurde der Versuch beendet. Die Auswertung der Versuche erfolgte durch Vergleich der Kontrollgruppen mit den behandelten Gruppen. Hierbei wurde für die Wirksamkeit der Substanzen sowohl die reduzierte Absterberate als auch die Verlängerung der Anfangsphase der Infektion als Kriterium herangezogen.

Die Verbindung des Beispiels 9 zeigte sowohl eine Verlängerung der Anfangsphase der Infektion als auch eine Reduzierung der Absterberate. Die Effekte wurden im Konzentrationsbereich von 0,1 bis 1 mg/kg Körpergewicht

beobachtet. Die Verbindung des Beispiels 16 zeigte im Konzentrationsbereich von 1-10 mg/kg Körpergewicht dosisabhängig eine Reduktion der Absterberate.

Die neuen Verbindungen können somit dazu benutzt werden, bereits unterschwellig ablaufende Abwehrreaktionen bei Mensch und Tier zu fördern. Die Verbindungen eignen sich demnach besonders für die Stimulation der körpereigenen Abwehr, z.B. bei chronischen und akuten Infektionen, sowie bei angeborenen, aber auch erworbenen allgemeinen (d.h. nicht antigenspezifischen) immunologischen Defektzuständen, wie sie im Alter, im Verlauf schwerer Primärerkrankungen und vor allem nach Therapie mit ionisierenden Strahlen oder mit Immunsuppressiv wirkenden Stoffen auftreten. Die genannten Stoffe können somit vorzugsweise auch in Kombination mit antiinfektiösen Antibiotika, Chemotherapeutika oder anderen Heilverfahren verabreicht werden, um immunologischen Schädigungen entgegenzuwirken. Schließlich sind die beschriebenen Stoffe auch zur allgemeinen Prophylaxe von Infektionskrankheiten bei Mensch und Tier geeignet.

Die pharmazeutischen Präparate der vorliegenden Erfindung sind vorzugsweise Tabletten oder Gelatinekapseln, welche die Wirkstoffe zusammen mit Verdünnungsmitteln, z.B. Laktose, Mannit, Sorbit, Cellulose und/oder Schmiermitteln, z.B. Kieselerde, Talk, Stearinsäure oder Salze davon, wie Magnesium- oder Calciumstearat, und/oder Polyethylenglykol, enthalten. Tabletten enthalten ebenfalls

Bindemittel, z.B. Magnesiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht, Sprengmittel, z.B. Stärken, Agar, Alginsäure oder ein Salz davon, wie Natriumalginat und/oder Brausemischungen, oder Adsorptionsmittel, Farbstoffe, Geschmackstoffe und Süßmittel. Injizierbare Präparate sind vorzugsweise isotonische wäßrige Lösungen oder Suspensionen. Suppositorien, Salben oder Cremen sind in erster Linie Fettemulsionen oder -suspensionen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1 % bis etwa 75 % insbesondere von etwa 1 % bis 50 % der genannten Aktivstoffe.

Die oral applizierten Präparate der vorliegenden Erfindung können auch mit einem gegen Magensaft beständigen Überzug versehen werden.

Die erfindungsgemäßen Verbindungen können als abwehrsteigernde und immunpotenzierende Mittel zur Behandlung von chronischen und akuten Infektionen (z.B. bakterielle, virale und parasitäre) verwendet werden. Sie können

ebenfalls als Adjuvantien bei Stimulierung von Phagozytose, bei Dysregulationen des Abwehr- und Immunsystems verwendet werden.

## Beispiele

1. 1-Desoxy-1-butylamino-D-fructose-Oxalat

18 g D-Glucose werden in 200 ml Dioxan und 100 ml Ethanol aufgeschlämmt und mit 7,3 g Butylamin versetzt. Die Mischung wird auf 70°C erwärmt und nach 10 min mit wasserfreier Oxalsäure versetzt. Die Temperatur wird 4 h beibehalten. Nach dem Abkühlen auf Raumtemperatur kristallisiert das Produkt aus. Der Feststoff wird abgesaugt, fünfmal mit je 50 ml Ethanol nachgewaschen und i.Vak. getrocknet.

2. 1-Desoxy-1-dodecylamino-D-fructose-Oxalat

Herstellung analog zu Beispiel 1 aus 18 g D-Glucose und 18,5 g Dodecylamin.

3. 1-Desoxy-1-tetradecylamino-D-fructose-Oxalat

Herstellung analog Beispiel 1 aus 18 g D-Glucose und 21,3 g Tetradecylamin.

4. 1-Desoxy-1-octadecylamino-D-fructose-Oxalat

Herstellung analog Beispiel 1 aus 18 g D-Glucose und 27 g Stearylamin.

5. <u>1-Desoxy-1-dodecylamino-D-tagatose-Oxalat</u>

Herstellung analog Beispiel 1 aus 18 g D-Galactose und 18,5 g Dodecylamin.

6. <u>1-Desoxy-1-tetradecylamino-D-tagatose-Oxalat</u>

Herstellung analog Beispiel 1 aus 18 g D-Galactose und 21,3 g Tetradecylamin.

7. <u>1-Desoxy-1-octadecylamino-D-tagatose-Oxalat</u>

Herstellung analog Beispiel 1 aus 18 g D-Galactose und 27 g Stearylamin.

8. <u>1-Desoxy-1-(N-butyl-dodecanoylamido)-D-fructose</u>

5 g der Verbindung aus Beispiel 1 werden in 80 ml Tetrahydrofuran aufgeschlämmt und mit 4,3 g Dodecansäurechlorid versetzt. Nach beendeter Reaktion wird von Feststoff abgesaugt, das Filtrat zum Sirup angedampft und säulenchromatographisch gereinigt. (Laufmittel Dichlormethan/Methanol 20/1).

Rf-Wert 0,28 in Toluol/n-Propanol 6/1
$\alpha_D$ = - 15°C (c = 1,0 in Dichlormethan)

9. <u>1-Desoxy-1-(N-dodecyl-octadecanoylamido)-D-fructose</u>

Herstellung analog Beispiel 8 aus 6,7 g der Verbindung aus Beispiel 2 und 6,0 g Stearinsäurechlorid.

Rf-Wert 0,31 in Toluol/n-Propanol 6/1
$\alpha_D$ = -8,5° (c = 1,0 in Dichlormethan).

10. 1-Desoxy-1-(N-dodecyl-dodecanoylamido)-D-fructose

Herstellung analog Beispiel 8 aus 6,7 g der Verbindung aus Beispiel 2 und 4,3 g Dodecansäurechlorid.

$\alpha_D$ = -11,3° (c = 1,0 in Dichlormethan)

11. 1-Desoxy-1-(N-dodecyl-hexadecanoylamido)-D-fructose

Herstellung analog Beispiel 8 aus 6,7 g der Verbindung aus Beispiel 2 und 5,4 g Hexadecansäurechlorid.

$\alpha_D$ = -9,3° (c = 1,1 in Dichlormethan)

12. 1-Desoxy-1-(N-tetradecyl-hexadecanoylamido)-D-fructose

Herstellung analog Beispiel 8 aus 7,2 g der Verbindung aus Beispiel 3 und 5,4 g Hexadecansäurechlorid.

$\alpha_D$ = -8,7° (c = 1,05 in Dichlormethan)

0152856

13. 1-Desoxy-1-(N-tetradecyl-octadecanoylamido)-D-fructose

Herstellung analog Beispiel 8 aus 7,2 g der Verbindung aus Beispiel 3 und 6,0 g Octadecansäurechlorid.

$\alpha_D$ = -4,6° (c = 1,05 in Dichlormethan)

14. 1-Desoxy-1-(N-tetradecyl-ölsäureamido)-D-fructose

Herstellung analog Beispiel 8 aus 7,2 g der Verbindung aus Beispiel 3 und 6,0 g Ölsäurechlorid.

$\alpha_D$ = -4,7° (c = 1,80 in Dichlormethan)

15. 1-Desoxy-1-(N-octadeyl-dodecansäureamido)-D-fructose

Herstellung analog Beispiel 8 aus 8,0 g der Verbindung aus Beispiel 4 und 4,3 g Dodecansäurechlorid.

$\alpha_D$ = -5,6° (c = 2,04 in Dichlormethan)

16. 1-Desoxy-1-(N-octadecyl-octadecansäureamido)-D-fructose

Herstellung analog Beispiel 8 aus 8,0 g der Verbindung aus Beispiel 4 und 6,0 g Octadecansäurechlorid

$\alpha_D$ = -2,8° (c = 0,95 in Dichlormethan)

17. 1-Desoxy-1-(N-tetradecyl-N-dodecyloxycarbonyl)-amino-D-fructose

Herstellung analog Beispiel 8 aus 7,2 g der Verbindung aus Beispiel 3 und 4,7 g Chlorameisensäuredodecylester.

$\alpha_D$ = -6,9° (c = o, 85 in Dichlormethan)

18. 1-Desoxy-1-(N-tetradecyl-N-octadecyloxycarbonyl)-amino-D-fructose

Herstellung analog Beispiel 8 aus 7,2 g der Verbindung aus Beispiel 3 und 6,4 g Chlorameisensäureoctadecylester

$\alpha_D$ = -3,2° (c = 1,15 in Dichlormethan)

19. 1-Desoxy-1-(N-Octadecyloxycarbonyl)-amino-D-fructose

Herstellung analog Beispiel 8 aus 8,0 g der Verbindung aus Beispiel 4 und 6,4 g Chlorameisensäureoctadecylester

$\alpha_D$ = -2,6° (c = 0,95 in Dichlormethan)

20. **1-Desoxy-1-[N-dodecyl-N-(dodecylamino-carbonyl)]-amino-D-fructose**

Herstellung analog Beispiel 8 aus 6,7 g der Verbindung aus Beispiel 2 und 3,9 g Dodecylisocyanat

$\alpha_D$ = -4,6° (c = 1,20 in Dichlormethan)

21. **1-Desoxy-1-[N-dodecyl-N-(octadecylaminocarbonyl)]-amino-D-fructose**

Herstellung analog Beispiel 8 aus 6,7 g der Verbindung aus Beispiel 2 und 5,6 g Octadecylisocyanat.

$\alpha_D$ = -3,8° (c = 1,0 in Dichlormethan)

22. **1-Desoxy-1-[N-octadecyl-N-(dodecylaminocarbonyl)]-amino-D-fructose**

Herstellung analog Beispiel 2 aus 8,0 g der Verbindung aus Beispiel 4 und 3,9 g Dodecylisocyanat

$\alpha_D$ = -4,3° (c = 1,05 in Dichlormethan)

23. **1-Desoxy-1-[N-octadecyl-N-(octadecylaminocarbonyl)]-amino-D-fructose**

Herstellung analog Beispiel 8 aus 8 g der Verbindung aus Beispiel 4 und 5,6 g Octadecylisocyanat.

$\alpha_D$ = -1,9° (c = 0,83 in Dichlormethan)

24. <u>1-Desoxy-1-(N-dodecyl-dodecanoylamido)-D-tagatose</u>

Herstellung analog Beispiel 8 aus 6,7 g der Verbindung aus Beispiel 5 und 4,3 g Dodecansäurechlorid.

$\alpha_D$ = -10,4° (c = 0,56 in Dichlormethan)

25. <u>1-Desoxy-1-(N-dodecyl-octadecanoylamido)-D-tagatose</u>

Herstellung analog Beispiel 8 aus 6,7 g der Verbindung aus Beispiel 5 und 6,0 g Octadecansäurechlorid.

$\alpha_D$ = -3,6° (c = 0,95 in Dichlormethan)

26. <u>1-Desoxy-1-(N-tetradeyl-octadecanoylamido)-D-tagatose</u>

Herstellung analog Beispiel 8 aus 7,2 g der Verbindung aus Beispiel 6 und 5,0 g Octadecansäurechlorid.

$\alpha_D$ = -3,4° (c = 1,0 in Dichlormethan)

27. <u>1-Desoxy-1-(N-octadecyl-dodecanoylamido)-D-tagatose</u>

Herstellung analog Beispiel 8 aus 8,0 g der Verbindung aus Beispiel 7 und 4,3 g Dodecansäurechlorid.

$\alpha_D$ = -3,6° (c = 1,1 in Dichlormethan)

0152856

28. <u>1-Desoxy-1-(N-octadecyl-octadecanoylamido)-D-tagatose</u>

Herstellung analog Beispiel 8 aus 8,0 g der Verbindungaus Beispiel 7 und 6,0 g Octadecansäurechlorid.

$\alpha_D$ = -3,4° (c = 0,85 in Dichlormethan)

29. <u>1-Desoxy-1-(N-octadecyl-N-tetradecyloxycarbonyl)-amino-D-tagatose</u>

Herstellung analog Beispiel 8 aus 8,0 g der Verbindung aus Beispiel 7 und 5,2 g Chlorameisensäure-tetradecylester.

$\alpha_D$ = -3,8° (c = 1,8 in Dichlormethan)

30. <u>1-Desoxy-1-(N-octadecyl-N-octadecylamino-carbonyl)-amino-D-tagatose</u>

Herstellung analog Beispiel 8 aus 8,0 g der Verbindung aus Beispiel 7 und 5,6 g Octadecylisocyanat

$\alpha_D$ = -3,2° (c = 1,0 in Dichlormethan)

31. <u>1-Desoxy-1-/N-dodecyl-N-(4-tert.-butylcyclohexyl-oxycarbonyl)7-amino-D-fructose</u>

Herstellung analog Beispiel 8 aus 6,7 g der Verbindung aus Beispiel 2 und 4,4 g Chlorameisensäure-4-tert.-butylcyclohexylester.

$[\alpha]_D$ = -1,9° (c = 1,05 in Dichlormethan)

32. 1-Desoxy-1-[N-octadecyl-N-(4-tert.-butyl-cyclohexyl-oxycarbonyl)]-amino-D-fructose

Herstellung analog Beispiel 8 aus 8,0 g der Verbindung aus Beispiel 4 und 4,4 g Chlorameisensäure-4-tert.-Butylcyclohexylester.

$[\alpha]_D$ = -2,2° (c = 1,05 in Dichlormethan)

Patentansprüche

1. Verbindungen der Formeln Ia bis Ic

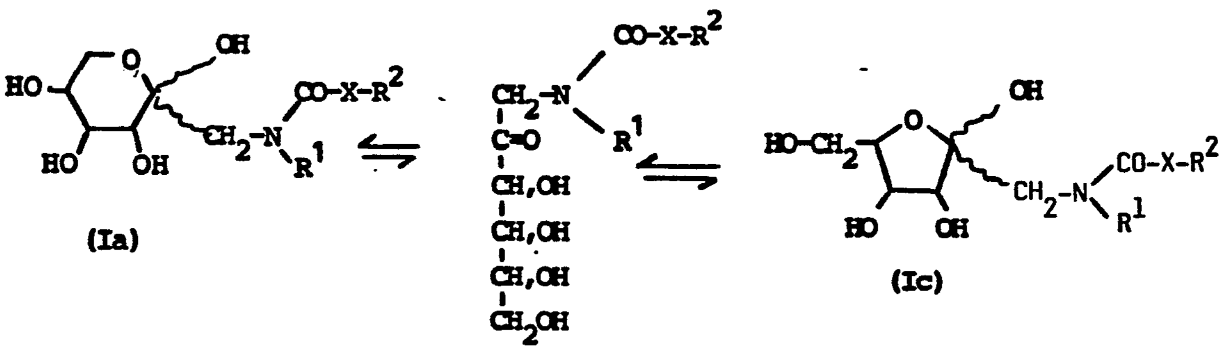

(Ia) (Ib) (Ic)

in denen

$R^1$ Wasserstoff oder einen gegebenenfalls substituierten, geradkettigen oder verzweigten Alkyl- oder ein- oder mehrfach ungesättigten Alkenylrest mit jeweils bis zu 30 C-Atomen darstellt,

X für $CH_2$, O, S oder NR steht, worin R Wasserstoff oder eine Alkylgruppe mit bis zu 20 C-Atomen bedeutet und

$R^2$ Wasserstoff oder einen gegebenenfalls substituierten, geradkettigen oder verzweigten, gesättigten oder ein- oder mehrfach ungesättigten Kohlenwasserstoffrest mit bis zu 30 C-Atomen darstellt.

2. Pharmazeutisch verträgliche Salze der Verbindungen nach Anspruch 1.

3. Verbindungen nach den Ansprüchen 1 oder 2, in denen

$R^1$ einen gegebenenfalls substituierten geradkettigen oder verzweigten Alkylrest mit 7 - 21 C-Atomen oder einen ein- oder zweifach ungesättigten, gegebenenfalls substituierten geradkettigen oder verzweigten Alkenylrest mit 7 - 21 C-Atomen darstellt.

4. Verbindungen nach den Ansprüchen 1-3, in denen $R^2$ 1-20 C-Atome aufweist.

5. Verbindungen der Ansprüche 1-4 zur Bekämpfung von Krankheiten.

6. Verfahren zur Herstellung der Verbindungen gemäß Anspruch 1, dadurch gekennzeichnet, daß man

a) eine Hexose mit einer Aminoverbindung der Formel $R^1-NH_2$ in der $R^1$ die in Anspruch 1 angegebene

Bedeutung hat zu einem Glykosylamin der Formel II

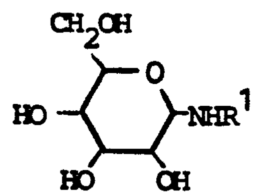

(II)

umsetzt,

b) dieses nach der Verfahrensweise einer Amadori-Umlagerung in eine N-alkylierte 1-Amino-1-desoxyketose der Formel III

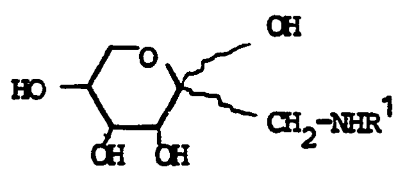

(III)

umlagert, und

c) in die Verbindungen III mit einem geeigneten Reagenz die Gruppe $-COX-R^2$ einführt, in der X und $R^2$ die in Anspruch 1 angegebene Bedeutung haben.

0152856

7. Verfahren nach Anspruch 6, dadurch gekennzeichnet, daß die Umsetzung gemäß Verfahrensschritt c)

- für Verbindungen, in denen X für $CH_2$ steht mit einem Reagenz der Formel $R^2-CH_2-CO-Y$ durchführt, worin $R^2$ die in Anspruch 1 angegebene Bedeutung hat und Y für Halogen oder einer in Amidierungsreaktionen üblichen Abgangsgruppe steht, oder

- für Verbindungen, in denen X für O steht, mit einem Reagenz der Formel $R^2-O-CO-Y'$ durchführt worin $R^2$ obengenannte Bedeutung hat und Y' für Halogen steht, oder

- für Verbindungen, in denen X für S steht, mit einem Reagenz der Formel $R^2-S-CO-Y'$ durchführt, wobei $R^2$ und Y' obengenannte Bedeutung haben, oder

- für Verbindungen, in denen X für NH steht mit einem Reagenz der Formel $R^2-NCO$ durchführt, worin $R^2$ die obengenannte Bedeutung hat, oder

- für Verbindungen, in denen X für NH oder N-R steht, worin R für eine Alkylgruppe mit bis zu 20 C-Atomen steht,

zunächst mit einem aromatischen Halogenameisensäureester zu einem Carbamat durchgeführt wird, das dann mit einem Amin der Formel $R^1-NH_2$ oder $R^2-NH-R$, worin R und $R^2$ die obengenannte Bedeutung haben, umgesetzt wird.

**8. Arzneimittel enthaltend mindestens eine Verbindung nach Anspruch 1.**

**9. Verwendung der Verbindungen nach den Ansprüchen 1-4 bei der Bekämpfung von Krankheiten.**

10. Verwendung der Verbindungen nach den Ansprüchen 1 - 4 zur Herstellung von Arzneimitteln.